# FASCICULE DE BREVET EUROPEEN

(11) **EP 0 843 562 B1**
(45) Date de publication et mention de la délivrance du brevet: **13.03.2002**
(21) Numéro de dépôt: 96927103.0
(22) Date de dépôt: 29.07.1996
(51) Int. Cl.: A61L 27/00

(54) **COMPOSITION POUR BIO-MATERIAU, PROCEDE DE PREPARATION**
BIOMATERIAL ZUSAMMENSETZUNG UND VERFAHREN ZU SEINER HERSTELLUNG
BIOMATERIAL COMPOSITION AND METHOD FOR PREPARING SAME

(30) Priorité: 07.08.1995 FR 9509582
(43) Date de publication de la demande: 27.05.1998
(73) Titulaire: CENTRE NATIONAL DE LA RECHERCHE SCIENTIFIQUE (CNRS), 75016 Paris (FR)
(72) Inventeur: DACULSI, Guy, F-44360 Vigneux-de-Bretagne (FR); WEISS, Pierre, F-44000 Nantes (FR); DUPRAZ, Anne, F-35600 Redon (FR); LAPKOWSKI, Mieczyslam, PL-41-804 Zabrze (PL)
(74) Mandataire: Jacobson, Claude
(86) Numéro de dépôt international: FR9601196
(87) Numéro de publication internationale: WO9705911

(56) Documents cités:
- EP-A- 0 511 868
- WO-A-86/01113
- WO-A-95/21634
- GB-A- 2 248 232
- PATENT ABSTRACTS OF JAPAN vol. 015, no. 128 (C-0818), 28 Mars 1991 & JP,A,03 011006 (SANKIN KOGYO KK), 18 Janvier 1991, cité dans la demande

## Description

L'invention concerne une composition injectable pour un bio-matériau de comblement de tissus organiques de soutien, destiné à générer une fonction de résorption/substitution.

De l'état de la technique sont connus des substituts osseux à base de particules de phosphate de calcium et d'une colle biologique.

Ainsi, G. DACULSI et al. ont décrit dans Ann. Oto. Rhino. Laryngol. 101:1992, l'efficacité d'une composition microporeuse biphasique de phosphate de calcium pour l'oblitération de la cavité mastoïdienne.

Les mêmes auteurs ont également rapporté l'efficacité d'une composition macroporeuse de phosphate. de calcium biphasique pour la réparation chirurgicale d'os longs (Journal of Biomedical Materials Research, Vol. 24, 379-396) et dans les arthrodèses vertébrales (Clinical Orthopadics and Related Research, 248, 1989, 169-175).

En odontologie, l'utilité des phosphates de calcium a été montrée dans plusieurs articles : A. Jean et col dans Cells and materials 1993;3:193-200. "Pulpal response to calcium phosphate materials. In vivo study of Calcium Phosphate materials in Endodontics" ; B.Al-liot-Licht et col dans Arch. Oral Biol. 1994;39:481-489 "Comparative effect of calcium hydroxide and hydroxyapatite on the cellular activity of human pulpal fibroblasts. An in vivo approach."

D'autre part, JP 3 011 006 décrit un cément pour tissus durs comprenant une phase minérale constituée d'au moins 60% de phosphate tri-calcique alpha et de l'hydroxyapatite et/ou un mono-phosphate de calcium ; et une phase liquide comprenant de la carboxyméthylcellulose.

Une telle composition présente cependant l'inconvénient, en raison de la solubilité trop grande du phosphate tri-calcique α, de ne pas être suffisamment stable pour permettre un processus de résorption/substitution du tissu dur. En outre, une telle composition est susceptible de générer des processus inflammatoires néfastes. En outre, ce mélange constitue un ionomère de calcium impropre à l'injection après quelques minutes par durcissement du mélange dès sa constitution. Cette association présente une double instabilité, une contraction volumétrique avec relarguage d'eau après plusieurs jours, et surtout une chute de la viscosité après stérilisation à l'autoclave du mélange. Elle ne permet pas la réalisation d'un matériau "prêt à l'emploi", stérile, injectable.

La présente invention s'est fixé pour but de fournir une composition de bio-matériau chargée en phase minérale, réhabitable, injectable, et durcissant in situ dans le lieu d'implantation.

En particulier, cette composition doit présenter les propriétés suivantes :
- Elle doit être stérilisable.
- Elle ne doit pas être toxique in vivo.
- Elle doit posséder une forte charge minérale induisant un front de minéralisation et/ou une cicatrisation tissulaire.
- Elle doit comprendre un agent de dispersion ayant un rôle de vecteur qui transporte la charge minérale dans le site opératoire, puis qui la maintient dans ce site jusqu'à la cicatrisation tissulaire en jouant un rôle de matrice pour matériau composite.
- Elle doit pouvoir être introduite dans un milieu biologique, notamment par injection via une seringue ou un appareil de type "lentulo" utilisé en chirurgie dentaire (composition dite lentulable), à l'état fluide ou pâteux, avant de durcir au contact avec les fluides biologiques tamponnés.
- Elle doit être stable pour être stockable relativement longtemps avant l'emploi.
- Elle doit être facile d'emploi.

Ce but a été atteint par la présente invention, dont l'objet est une composition pour bio-matériau de résorption/substitution de tissus organiques de soutien, comprenant :
- 20 à 75 % en poids d'une phase minérale constituée par des particules comprentant soit de l'hydroxyapatite (A), éventuellement en mélange avec du phosphate tricalcique β (B), soit du calcium-titane-phosphate (Ca(Ti)₄(PO₄)₆) (C), et
- 80 à 25% en poids d'une phase liquider comprenant une solution aqueuse d'un polymère biocompatible, hydrosoluble, autoréticulable sous l'effet du pH du milieu.

La phase minérale à base de particules de phosphate(s) de calcium fournit la charge minérale nécessaire au front de minéralisation.

Le calcium-titane-phosphate (CTP) de formule Ca(Ti)₄(PO₄)₆ est de préférence du type Calci-Métallo-phosphate "Nasicon-like".

Les particules minérales comprennent avantageusement un mélange d'hydroxyapatite (A) et de phosphate tricalcique β (B), ces mélanges étant communément désignés par le sigle BCP (Biphasic Calcium Phosphate). Le mélange comprend de préférence les composés A et B dans un rapport pondéral A/B compris entre 80/20 et 30/70, en particulier d'environ 60/40.

Ce dernier type de charge consiste en un fritté haute température, broyé et calibré en poudre ou en granulés dont les particules ont un diamètre de 80 µm à 200 µm lors de l'élaboration de la composition. D'autres types de synthèse des charges ou d'autres granulométries peuvent toutefois être choisies en fonction des tissus et des indications. Le choix du diamètre particulaire peut être guidé en particulier par la cinétique de résorption et la rhéologie souhaitées. Des particules de diamètre inférieur à 80 µm ont en général une cinétique de résorption assez rapide. Elles trouvent leur application notamment en chirurgie orthopédique. Pour des applications endodontiques en chirurgie dentaire, les particules de diamètre supérieur à 200 µm sont à éviter car elles posent des problèmes à l'injection dus à la rhéologie de la composition.

D'autres charges minérales peuvent également être utilisées telles que les céramiques "Nasicon-like" CZP, les céramiques phosphocalciques ou les bioverres.

La phase liquide tient lieu d'une part d'agent de dispersion pour la charge minérale particulaire, apte à se mélanger intimement avec cette dernière, la composition finale pouvant se présenter sous une forme liquide ou plus ou moins pâteuse. Elle est d'autre part destinée à former une matrice de matériau composite incorporant la charge minérale.

A cet effet, la phase liquide comprend un polymère autoréticulable en solution aqueuse.

Compte tenu de l'application à un bio-matériau, le polymère doit être biocompatible, c'est-à-dire qu'il ne doit pas être toxique et ne doit pas provoquer de réaction de rejet lors de son implantation dans l'organisme. Par ailleurs, sa réticulation ne doit pas libérer de sous-produit toxique.

En outre, le polymère doit être stable à l'état non-réticulé dans un milieu donné et autoréticulable par simple mise en contact avec le milieu biologique dans lequel il est implanté. Les polymères qui remplissent le mieux ce critère sont ceux qui sont réticulables sous l'effet du pH des fluides biologiques tamponnés. De préférence, ces polymères sont solubles à l'état non-réticulé dans une phase aqueuse relativement basique et se réticulent sous l'effet d'une baisse de pH.

Il est particulièrement avantageux que le polymère réticule de façon covalente dans le milieu d'implantation pour produire un bio-matériau réticulé résistant.

Ainsi, on préfère les polymères qui sont susceptibles de subir, au pH du milieu, une réaction chimique conduisant à la formation de liaisons covalentes intermoléculaires et éventuellement intramoléculaires.

De tels polymères peuvent être choisis avantageusement parmi les polymères comportant des groupes latéraux contenant des fonctions réactives à base de silicium, tels que des groupes silanolates de métal alcalin ou d'ammonium, ou bien des groupes organosiliciés hydrolysables dans le milieu biologique pour former des silanolates sans libérer de produit toxique comme sous-produit d'hydrolyse.

Un groupe latéral silanolate approprié peut être par exemple un groupe de formule

-CH₂-CHOH-CH₂O- ( CH₂)₃-Si-( ONa)₃.

Pour fournir un taux de réticulation convenable pour l'application à un bio-matériau durcissant, il est souhaitable que les groupes latéraux porteurs de silicium, de type silanolate ou précurseur de silanolate, représentent de 1 à 5 % du poids sec total dudit polymère.

Le polymère de base auquel sont liés les groupes latéraux peut être de nature diverse à condition d'être biocompatible.

On peut le choisir avantageusement parmi la cellulose et les polymères dérivés de cellulose, dont la biocompatibilité est bien connue et appliquée en galénique aux matrices retard pour médicament. On peut citer notamment les éthers de cellulose non-ioniques, par exemple l'hydroxyéthylcellulose, l'hydroxyéthylméthylcellulose, ou l'hydroxypropylméthylcellulose.

Ainsi, un polymère autoréticulable par liaisons covalentes et dérivé de cellulose peut être obtenu par éthérification de cellulose ou d'un dérivé de celle-ci par réaction avec un composé de formule (1)

X Si(OZ)₃ (1)

où X représente un atome d'halogène ou un groupe hydrocarboné à fonction époxy, notamment en C₂₋₂₀, et Z est choisi parmi un atome d'hydrogène, un métal alcalin et un groupe alkyle, notamment en C₁₋₅.

Le composé de formule (1) peut être par exemple le (3-glycidoxypropyl)triméthoxysilane

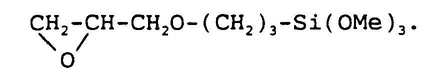

La synthèse de polymères dérivés d'hydroxyéthylcellulose (HEC) et de (3-glycidoxypropyl)triméthoxysilane est décrite par Arjun C. Sau et Thomas G. Majewicz dans Cellulose Ethers ; Self-cross-linking mixed ether silyl derivatives, ACS Symp. Ser. 1992, Vol 476, 265-72.

En milieu basique, le composé organosilicié se greffe sur l'HEC avec ouverture de l'époxyde et les groupes méthoxysilane sont hydrolysés pour conduire à un polymère répondant à la formule simplifiée : 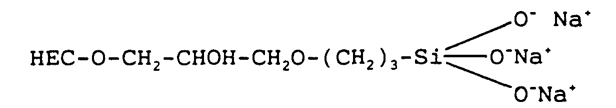 Ce polymère est stable en solution aqueuse à un pH supérieur ou égal à environ 10,5. Une acidification de la solution provoque une augmentation progressive de la viscosité et la formation d'un hydrogel. Ce phénomène physique correspond à la réticulation du polymère par (i) transformation des groupes silanolates en groupes silanols : 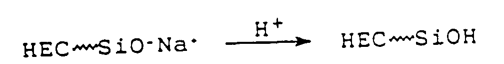 puis, formation d'un réseau tridimensionnel par (ii) condensation d'un silanol sur un autre silanol 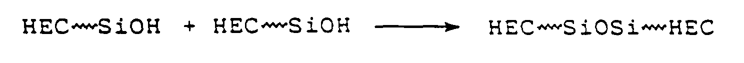 et/ou
(iii) condensation d'un silanol sur un groupe hydroxy des cycles des éthers de cellulose ou des substituants

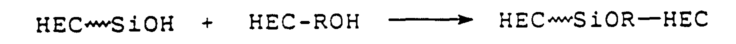

Cette réticulation de type covalent provoquée par une baisse du pH de la solution aqueuse du polymère est réversible et l'hydrogel se redissout lorsque l'on augmente le pH du milieu.

Un tel polymère peut se trouver à l'état dissous dans une composition selon l'invention contenant du BCP, grâce à la basicité de ces mélanges de phosphates de calcium. La composition peut également contenir une base de tout type pour assurer l'alcalinité nécessaire à la solubilisation du polymère.

Dans la composition selon l'invention, une réticulation auxiliaire de type ionique peut également intervenir au niveau des groupes silanolates pontés via les cations divalents Ca²⁺.

La fonctionnalisation par des groupes silanolates autoréticulables peut s'appliquer à tout autre type de polymère hydrosoluble et biocompatible, présentant une réactivité adéquate. On peut citer d'autres polysaccharides notamment le guar, l'amidon et leurs dérivés éthérifiés.

La composition selon l'invention constitue, du fait de ses deux composants, un système présentant une forte charge minérale, auto-durcissant au contact des milieux biologiques tamponnés, sans système adjuvant ou catalytique de pontage, la réticulation du polymère étant déclenchée par la modification du pH de la composition.

Le matériau réticulé obtenu sera plus ou moins dense en fonction de la teneur en polymère et de la quantité de fonctions réticulables dans ledit polymère.

La phase liquide est également déterminante eu égard aux propriétés rhéologiques et donc à la viscoélasticité de la composition finale non réticulée destinée à être implantée dans le milieu biologique.

A cet effet, la concentration en polymère autoréticulable est avantageusement comprise entre 1 et 5% en poids, de préférence d'environ 2% en poids par rapport au poids de la phase liquide.

En variante, la phase liquide de la composition peut comprendre en outre un polymère biocompatible non réticulable, ce qui permet de fixer indépendamment les propriétés rhéologiques de la composition avant injection et le degré de durcissement du matériau implanté dans le milieu. Tout polymère biocompatible hydrosoluble peut être utilisé à cet effet, notamment les polysaccharides ou dérivés tel que la cellulose et les éthers de cellulose. Les proportions relatives des polymères seront ajustées de façon classique en fonction des propriétés désirées.

La composition selon l'invention est obtenue par mélange des constituants de la phase minérale et de la phase liquide.

Les granulés ou la poudre de phosphate tricalcique β et d'hydroxyapatite ou de CTP de la phase minérale peuvent être obtenus comme décrit par DACULSI et al. (Rev. Chir. Orthop., 1989, 75(2) : 65-71).

Un intérêt majeur de la composition selon l'invention est qu'elle est stérilisable, avant ou après le mélange des deux phases.

L'invention a donc également pour objet une composition telle que décrite précédemment et stérile.

Une stérilisation classique à l'oxyde d'éthylène n'est pas possible pour un tel matériau "prêt à l'emploi". Il faut en effet stériliser sous leur forme sèche les composants du mélange ce qui impose une manipulation pour le chirurgien avant l'injection. Cette manipulation est difficile et non reproductible.

Selon l'invention, la préparation d'une composition stérile est réalisée par dissolution du constituant polymère de la phase liquide dans de l'eau à une viscosité déterminée en fonction de la viscosité finale souhaitée. La solution obtenue est ensuite: mélangée avec la phase minérale et la composition résultante introduite dans des flacons de conditionnements scellés et stérilisés à l'autoclave à 121°C pendant 20 minutes.

La stérilisation par autoclave du mélange peut également, suivant les exigences, être effectuée à une température de 130°C pendant 30 minutes, si le potentiel de dissolution de la charge n'est pas trop important.

La viscosité initiale de la composition (concentration en polymère) doit être adaptée pour obtenir la viscosité désirée après stérilisation, c'est-à-dire la concentration en polymère telle que définie ci-dessus.

Si le potentiel de dissolution de la charge phosphocalcique ne permet pas la stérilisation du mélange à 121 ou 130°C pour cause de problèmes rhéologiques à cette température, la phase liquide aqueuse est stérilisée à l'autoclave séparément de la charge dans l'une des conditions ci-dessus, puis le mélange des deux parties stériles se fait en chambre blanche stérile.

L'invention concerne également un nécessaire pour la préparation d'une composition pour bio-matériau, comprenant, d'une part, une phase minérale stérile et, d'autre part, une phase liquide stérile, telles que décrites précédemment, destinées à être mélangées extemporanément sous atmosphère stérile avant implantation.

La composition selon l'invention est stockable sous sa forme mélangée prête à l'emploi ou dissociée prête au mélange sans perte notable de qualité.

La composition selon l'invention peut être utilisée comme matériau de comblement osseux de tissus organiques de soutien de l'organisme, ce matériau étant destiné à générer une fonction de résorption/substitution. Elle peut notamment être utilisée comme matériau de comblement associé à des implants ou des prothèses articulaires, ou pour toute application et indication chirurgicale nécessitant une certaine "tenue" ou des propriétés mécaniques.

L'invention a donc également pour objet un procédé du traitement du corps humain ou animal, comprenant l'administration par injection d'une composition selon l'invention en un site normalement occupé par un tissu organique de soutien, pour générer une fonction de résorption/substitution de ce tissu.

Un exemple d'application est la chirurgie dentaire. L'injection peut être réalisée à l'aide d'un système comportant une seringue stérilisable et des embouts munis de pistons à usage unique, par exemple le système commercialisé par HAWE NEOS DENTAL, comprenant une seringue stérilisée à l'autoclave (Réf. N° 440, Seringue Hawe-Centrix C-R ^{R} , Mark III) et des embouts (Réf. N° 445).

Un autre exemple d'application de la composition est la chirurgie orthopédique, où elle peut être injectée notamment pour traiter les problèmes d'instabilité du rachis lombaire, ou en tant que matériau de comblement (agent de scellement) associé à une prothèse articulaire, par exemple une prothèse de hanche. La composition peut être injectée par voie percutanée.

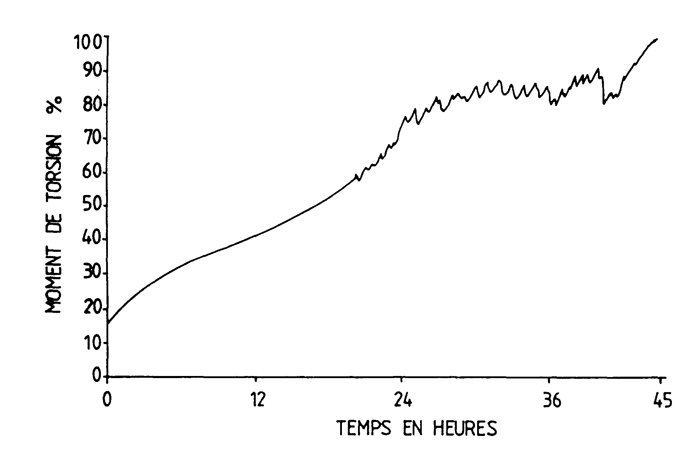

On donnera ci-après un exemple de composition selon l'invention, dont les propriétés sont illustrées en particulier sur la figure unique qui présente la courbe de durcissement de la composition en fonction du temps dans un milieu tamponné à pH 7 à 25°C.

### EXEMPLE DE COMPOSITION

Un polymère autoréticulable est préparé à partir d'un éther non-ionique de cellulose de la façon. suivante :
Dans un réacteur sous agitation magnétique, équipé d'un réfrigérant, on installe une atmosphère neutre d'azote et on introduit 1 l de propanol, 20 g d'hydroxyéthylcellulose de haut poids moléculaire (qui est insoluble dans le propanol), et 1 g de soude. Après une heure d'agitation, on ajoute à la suspension de polymère cellulosique 0,4 g de 3-glycidoxypropyltriméthoxysilane (soit 2 % en poids par rapport au poids de polymère).

Le mélange est chauffé 1 h jusqu'à 80°C, puis 2h au reflux à environ 110°C.

L'agitation est maintenue une nuit sans chauffer, puis le polymère est filtré puis lavé au propanol, à l'acide acétique, de nouveau au propanol et enfin à l'acétone.

Le polymère silanisé est solubilisé dans une solution aqueuse de soude décimolaire (pH=13) pendant 3 jours, à raison de 2 % en poids de polymère sec dans le solvant aqueux.

30 g de cette phase liquide sont mélangés avec 20 g d'un mélange de 40 % de phosphate tricalcique β et de 60 % d'hydroxyapatite.

Ce mélange est déposé dans du papier filtre, ayant la forme d'un tube fermé à une extrémité, habituellement utilisé pour un extracteur.

Le filtre rempli du mélange est mis en place dans une cuve contenant une solution tampon de NaCl 9 %, de pH=7 à une température de 25°C, modélisant un milieu biologique. Le filtre permet la diffusion ionique entre le mélange et la solution tampon.

Le durcissement de la composition dû à la chute de pH est suivi par la mesure de l'évolution de la viscosité du mélange au cours du temps, au moyen d'un viscosimètre Brookfield, muni d'une aiguille n° 29 pour petits échantillons, avec une vitesse de cisaillement lente de 1 tour par minute pour éviter la rupture du gel en formation.

La courbe de la figure unique présente l'évolution du moment de torsion du ressort du viscosimètre (mesurant l'évolution de la contrainte de cisaillement) en fonction du temps.

Elle montre que le mélange durcit spontanément à pH 7 de façon progressive. La viscosité initiale étant de 141 Pa.s, elle atteint 1 000 Pa.s au bout de 41 heures, alors que le pH de la solution tampon n'a pas évolué.

La composition préparée dans cet exemple est stérilisable à l'autoclave à 121°C pendant 20 minutes.

### ESSAI DE TOXICITE

Andriano K.P. et col dans un article du Journal of Applied Biomaterials Vol. 3, 197-206 (1992) a montré la non cytotoxicité des silanes par contact direct de fibres minérales silanisées avec des cellules de fibroblastes de souris L929.

On vérifie également que l'hydroxyéthylcellulose modifiée par l'époxysilane n'est pas cytotoxique. En utilisant des cellules L 929, le dépôt direct des cellules sur le polymère silanisé et séché en film montre 97 à 99 % de cellules vivantes après 24 heures de contact direct.

Dans un essai indirect (transwell) où les cellules sont déposées sur une couche de gel, et le polymère est placé sur l'autre face de la couche gélifiée, on retrouve aussi 97 à 98 % de cellules vivantes après 24 heures, ce qui montre que les produits de diffusion à travers le gel ne sont pas toxiques.

Le polymère silanisé n'est pas cytotoxique suivant la norme Afnor NF S 90 702.

## Revendications

1. Composition pour bio-matériau de résorption/substitution de tissus organiques de soutien, comprenant :
- 20 à 75% en poids d'une phase minérale constituée par des particules comprenant soit de l'hydroxyapatite (A), éventuellement en mélange avec du phosphate tricalcique β (B), soit du calcium-titane-phosphate (Ca(Ti)₄ (PO₄)₆) (C), et
- 80 à 25% en poids d'une phase liquide comprenant une solution aqueuse d'un polymère biocompatible, hydrosoluble, autoréticulable sous l'effet du pH du milieu.

2. Composition selon la revendication 1, **caractérisée en ce que** la phase minérale comprend un mélange d'hydroxyapatite (A) et de phosphate tricalcique β (B) dans un rapport pondéral A/B de 80/20 à 30/70, notamment d'environ 60/40.

3. Composition selon la revendication 1 ou 2, **caractérisée en ce que** la phase minérale est constituée d'une poudre dont la granulométrie lors de l'élaboration de la composition est comprise entre 80 et 200 µm de diamètre.

4. Composition selon l'une quelconque des revendications précédentes, **caractérisée en ce que** ledit polymère s'autoréticule en formant des liaisons covalentes sous l'effet du pH.

5. Composition selon l'une quelconque des revendications précédentes, **caractérisée en ce que** ledit polymère comporte des groupes latéraux de type silanolate ou précurseurs de silanolate de métal alcalin ou d'ammonium.

6. Composition selon la revendication 5, **caractérisée en ce que** les groupes latéraux représentent de 1 à 5 % du poids sec total dudit polymère.

7. Composition selon l'une quelconque des revendications précédentes, **caractérisée en ce que** ledit polymère est dérivé d'un polymère choisi parmi la cellulose, un éther de cellulose non ionique, le guar et l'amidon.

8. Composition selon la revendication 7, **caractérisée en ce que** ledit polymère résulte de l'éthérification de cellulose ou d'un dérivé de celle-ci par un composé de formule (1) XSi(OZ)₃ où X représente un atome d'halogène ou un groupe hydrocarboné à fonction époxy, et Z est choisi parmi un atome d'hydrogène, un métal alcalin et un groupe alkyle.

9. Composition selon l'une quelconque des revendications précédentes, **caractérisée en ce que** la concentration du polymère autoréticulable dans la phase liquide est de 1 à 5% en poids.

10. Composition selon l'une des revendications 1 à 9, **caractérisée en ce qu'**elle est stérile.

11. Procédé de préparation d'une composition selon la revendication 10, **caractérisé en ce qu'**il comprend :
- la préparation d'une phase liquide par dissolution dudit polymère dans un solvant aqueux,
- le mélange de la phase minérale avec la phase liquide, puis
- la stérilisation du mélange ainsi obtenu.

12. Procédé selon la revendication 11, **caractérisé en ce que** l'étape de stérilisation est effectuée en autoclave à une température de 121 ou 130°C.

13. Nécessaire pour la préparation d'une composition stérile selon la revendication 10, **caractérisé en ce qu'**il comprend :
- une phase minérale stérile constituée par des particules comprenant soit de l'hydroxyapatite (A), éventuellement en mélange avec du phosphate tricalcique β (B), soit du calcium-titane-phosphate (Ca(Ti)₄ (PO₄)₆) (C), et
- une phase liquide stérile comprenant une solution aqueuse d'un polymère biocompatible, hydrosoluble, autoréticulable sous l'effet du pH du milieu,
destinées à être mélangées extemporanément sous atmosphère stérile avant implantation de la composition.

## Claims

1. Composition for a biomaterial for the absorption/substitution of organic supporting tissues, comprising:
- 20 to 75% by weight of a mineral phase consisting of particles comprising either hydroxyapatite (A), optionally mixed with tricalcium phosphate β (B), or calcium-titanium phosphate (Ca(Ti)₄ (PO₄)₆) (C), and
- 80 to 25% by weight of a liquid phase comprising an aqueous solution of a biocompatible water-soluble polymer which is self-crosslinkable under the effect of the pH of the medium.

2. Composition according to claim 1, **characterised in that** the mineral phase comprises a mixture of hydroxyapatite (A) and tricalcium phosphate β (B) in a weight ratio A/B of 80/20 to 30/70, particularly about 60/40.

3. Composition according to claim 1 or 2,
**characterised in that** the mineral phase consists of a powder the particle size of which during the production of the composition is between 80 and 200 µm in diameter.

4. Composition according to any one of the preceding claims, **characterised in that** the polymer crosslinks by itself, forming covalent bonds, under the effect of the pH.

5. Composition according to any one of the preceding claims, **characterised in that** the polymer comprises side groups of the silanolate type or precursors of alkali metal silanolate or ammonium silanolate.

6. Composition according to claim 5, **characterised in that** the side groups represent 1 to 5% of the total dry weight of the polymer.

7. Composition according to any one of the preceding claims, **characterised in that** the polymer is derived from a polymer selected from among cellulose, a nonionic cellulose ether, guar and starch.

8. Composition according to claim 7, **characterised in that** the polymer results from the etherification of cellulose or a derivative thereof by a compound of formula (1) XSi(OZ)₃ where X denotes a halogen atom or a hydrocarbon group with an epoxy function, and Z is selected from among a hydrogen atom, an alkali metal and an alkyl group.

9. Composition according to any one of the preceding claims, **characterised in that** the concentration of the polymer which is self-crosslinkable in the liquid phase is from 1 to 5% by weight.

10. Composition according to one of claims 1 to 9, **characterised in that** it is sterile.

11. Process for preparing a composition according to claim 10, **characterised in that** it comprises:
- preparing a liquid phase by dissolving said polymer in an aqueous solvent,
- mixing the mineral phase with the liquid phase, then
- sterilising the mixture thus obtained.

12. Process according to claim 11, **characterised in that** the sterilisation step is carried out in an autoclave at a temperature of 121 or 130°C.

13. Kit for preparing a sterile composition according to claim 10, **characterised in that** it comprises:
- a sterile mineral phase consisting of particles comprising either hydroxyapatite (A), optionally mixed with tricalcium phosphate β (B), or calcium-titanium phosphate (Ca(Ti)₄(PO₄)₆) (C), and
- a sterile liquid phase comprising an aqueous solution of a biocompatible water-soluble polymer which is self-crosslinkable under the effect of the pH of the medium,
which are intended to be mixed extemporaneously in a sterile atmosphere before the composition is implanted.

## Patentansprüche

1. Zusammensetzung für ein Biomaterial zur Resorption/Substitution von organischen Stützgeweben, umfassend:
- 20 bis 75 Gew.-% einer mineralischen Phase, die sich zusammensetzt entweder aus Hydroxyapatit (A), gegebenenfalls in Mischung mit β-Trikalziumphosphat (B), oder aus Kalzium-Titan-Phosphat (Ca(Ti)₄(PO₄)₆) (C), und
- 80 bis 25 Gew.-% einer flüssigen Phase, umfassend eine wässrige Lösung eines biokompatiblen, wasserlöslichen Polymeren, das unter dem Einfluss des pH-Wertes des Mediums selbstvernetzend ist.

2. Zusammensetzung gemäß Anspruch 1, dadurch charakterisiert, dass die mineralische Phase eine Mischung aus Hydroxyapatit (A) und β-Trikalziumphosphat (B) in einem Gewichtsverhältnis A/B zwischen 80/20 und 30/70, insbesondere um 60/40 umfasst.

3. Zusammensetzung nach Anspruch 1 oder 2, dadurch charakterisiert, dass die mineralische Phase aus einem Pulver besteht, dessen Granulometrie zum Zeitpunkt der Bearbeitung der Mischung aus einem Durchmesser zwischen 80 und 200 um besteht.

4. Zusammensetzung nach einem der vorhergehenden Ansprüche, dadurch charakterisiert, dass das besagte Polymer sich unter der Wirkung des pH unter Ausbildung von kovalenten Bindungen selbst vernetzt.

5. Zusammensetzung nach einem der vorhergehenden Ansprüche, dadurch charakterisiert, dass das besagte Polymer seitliche Gruppen vom Typ Silanolat oder von Silanolatvorstufen von Alkalimetallen oder Ammonium enthält.

6. Zusammensetzung nach Anspruch 5, dadurch charakterisiert, dass die seitlichen Gruppen 1 bis 5 Gew.-% des gesamten Trockengewichts des besagten Polymeren ausmachen.

7. Zusammensetzung nach einem der vorhergehenden Ansprüche, dadurch charakterisiert, dass das besagte Polymer sich ableitet aus einem Polymeren, das ausgewählt ist aus Cellulose, einem nicht-ionischen Ether von Cellulose, Guar-Gummi und Stärke.

8. Zusammensetzung nach Anspruch 7, dadurch charakterisiert, dass das besagte Polymer aus der Veresterung von Cellulose oder eines Derivates davon resultiert mit einer Verbindung der Formel (1) XSi(OZ)₃, in der X ein Halogenatom oder eine Hydrocarbongruppe mit einer Epoxyfunktion (groupe hydrocarboné ä fonction époxy) darstellt, und Z ausgewählt ist aus einem Wasserstoffatom, einem Alkalimetal und einer Alkylgruppe.

9. Zusammensetzung nach einem der vorhergehenden Ansprüche, dadurch charakterisiert, dass die Konzentration des selbstvernetzenden Polymeren in der flüssigen Phase von 1 bis 5 Gew.-% beträgt.

10. Zusammensetzung nach einem der Ansprüche 1 bis 9, dadurch charakterisiert, dass sie steril ist.

11. Verfahren zur Herstellung einer Zusammensetzung nach Anspruch 10, dadurch charakterisiert, dass es umfasst:
- die Herstellung einer flüssigen Phase durch Auflösung des besagten Polymeren in einem wässrigen Lösungsmittel,
- die Mischung der mineralischen Phase mit der flüssigen Phase, dann
- die Sterilisierung der auf diese Weise erhaltenen Mischung.

12. Verfahren nach Anspruch 11, dadurch charakterisiert, dass der Schritt der Sterilisierung in einem Autoklaven bei einer Temperatur von 121 bis 130 °C durchgeführt wird.

13. Set ("Nécessaire") für die Herstellung einer sterilen Zusammensetzung nach Anspruch 10, dadurch charakterisiert, dass sie umfasst:
- eine sterile mineralische Phase, die sich zusammensetzt aus Teilchen, die entweder aus Hydroxyapatit (A), gegebenenfalls in Mischung mit β-Trikalziumphosphat (B), oder aus Kalzium-Titan-Phosphat (Ca(Ti)₄(PO₄)₆) (C) bestehen; und
- einer sterilen flüssigen Phase, die sich zusammensetzt aus einer wässrigen Lösung eines biokompatiblen, wasserlöslichen, unter der Wirkung des pH des Mediums selbstvernetzenden Polymeren,
das dazu bestimmt ist, unmittelbar vor der Implantierung der Zusammensetzung in einer sterilen Atmosphäre vermischt zu werden.
